# EUROPEAN PATENT SPECIFICATION

(11) **EP 1 621 598 B1**
(45) Date of publication and mention of the grant of the patent: **02.01.2008**
(21) Application number: 04103671.6
(22) Date of filing: 30.07.2004
(51) Int. Cl.: C09K 11/06, G03C 1/73, C07C 43/215, C07D 317/50, C07D 307/36, C07D 207/323

(54) **Divinylfluorenes.**
Divinylfluorene
Divinylfluorènes

(43) Date of publication of application: 01.02.2006
(73) Proprietor: Agfa Graphics N.V., 2640 Mortsel (BE)
(72) Inventor: Williamson, Alexander AGFA-GEVAERT, 2640, Mortsel (BE); Callant, Paul AGFA-GEVAERT, B-2640 Mortsel (BE)
(74) Representative: Goedeweeck, Rudi

(56) References cited:
- EP-A- 0 624 580
- US-A- 3 980 713
- US-A- 4 062 686
- US-A- 4 555 474
- PATENT ABSTRACTS OF JAPAN vol. 1995, no. 11, 26 December 1995 (1995-12-26) -& JP 07 196632 A (FUJI PHOTO FILM CO LTD), 1 August 1995 (1995-08-01)

## Description

### FIELD OF THE INVENTION

The present invention relates to new classes of divinylfluorene compounds, a new synthetic approach for divinylfluorene compounds and the use of said new compounds as optical brighteners, optical sensitizers and for electroluminescent materials and devices.

### BACKGROUND OF THE INVENTION

The fluorene ring system and its numbering is demonstrated with the following formula:

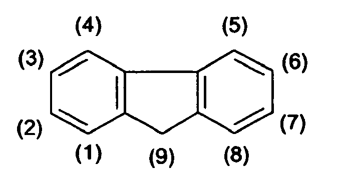

Commonly known divinylfluorene compounds belong to the following (2,7-distyryl) core structure (I):

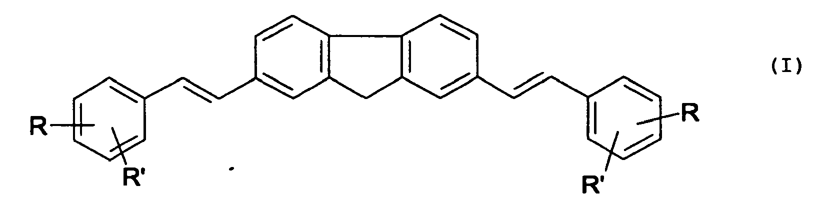

The unsubstituted compound of formula (I), wherein R and R' each represent a hydrogen atom and the disubstituted compounds, wherein both R and/or both R' represent a substituent selected from e.g. a hydroxy, alkoxy, dialkylamino, diarylamino or halogen group, are described in e.g. US 3 980 713, US 2003/091859, GB 2 313 127 and US 6 344 286.

For use as a luminescent material for organic and polymer-based electroluminescence elements, the distyryl fluorene compounds can also be polymerized or copolymerized as described e.g. in US 2003/091859.

Besides the aforementioned distyryl fluorene compounds (I) are also known the following di(vinyl hetaryl)fluorene compounds (F-1) and (F-2): 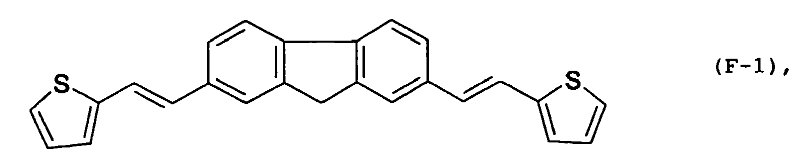 see e.g. Makromolekulare Chemie (1975), 176(3), 539-59,
and 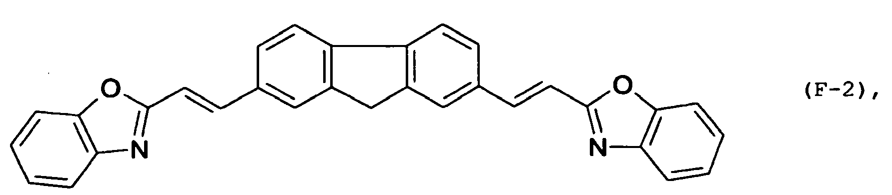 see e.g. JP 10 152 678 A2.

Apparently there is only known a very limited number of divinyl fluorene compounds from the prior art and such known compounds have drawbacks with respect to their properties when used as a) sensitizers for e.g. printing plate precursors, b) optical The synthetic method is disadvantageous, as the product can not easily be purified.

From A. Patra et al., Chemistry of Materials (2002), 14(10), 4044-4048, is known a Wittig reaction on only one side of the fluorene unit using Diphenylaminobenzaldehyde.

According to J.M. Kauffman and G. Moyna, Journal of Organic Chemistry (2003), 68(3), 839-853, 2,7-bis-phosphonate esters of fluorene can be reacted with p-substituted aldehydes to yield 2,7-distyrylfluorenes. The disadvantage of this method is that 5 reaction steps are necessary to obtain the 2,7-distyrylfluorenes from 9,9-dialkyl-2,7-dibromofluorene which itself must be prepared by alkylation of 2,7-dibromofluorene.

O. Mongin et al. describe in Tetrahedron Letters (2003), 44(44), 8121-8125 a synthetic method, wherein 9,9-dialkylfluorene is first converted to 2,7-dibromo-9,9-dialkylfluorene and then to 2,7-diformyl-9,9-dialkylfluorene. This is then reacted with [4-(dibutylamino)phenyl]methyltriphenylphosphonium bromide. The drawback is that arylmethyltriphenylphosphonium salts are not readily available, relative to the analogous aldehydes.

From the foregoing discussion it becomes evident that the known synthesis methods for divinylfluorene compounds are still unsatisfactory.

### SUMMARY OF THE INVENTION

It is an object of the present invention/ to find new divinylfluorene compounds that are suitable as e.g. optical brighteners, sensitizers for light sensitive materials or precursors for electroluminescent materials that do not show the drawbacks as discussed above.

It is also an object of the present invention to find a synthesis for divinylfluorene compounds that does not use many reaction steps, that yields very pure products without the necessity of difficult purifying steps and that only uses readily available reagents.

Further objects of the present invention are the use of the divinylfluorene compounds of the present invention as sensitizers for light sensitive materials, as optical brighteners and as monomers for the synthesis of polymeric light emitting materials of electroluminescent elements.

Preferred embodiments of the present invention are defined in the dependent claims.

### DETAILED DESCRIPTION OF THE INVENTION

The objects of the present invention are achieved by divinylfluorene compounds according to one of formulae (II) or (III): 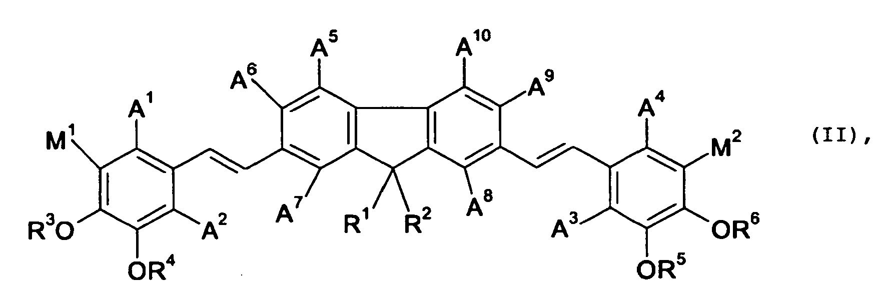 wherein
A¹ to A¹⁰ mutually independent mean a substituent selected from a non-metallic atom group,
M¹ means a hydrogen atom or OR⁷,
M² means a hydrogen atom or OR⁸,
R¹, R² mutually independent mean a substituent selected from a non-metallic atom group, and
R³ to R⁸ mutually independent mean a hydrogen atom, alkyl, an alkyl chain containing double or triple bonds, alkenyl, alkynyl, or any non-metallic atom group linked to the oxygen by a carbon atom, and wherein one or more pairs of said substituents can jointly mean the remaining atoms to form a ring; or 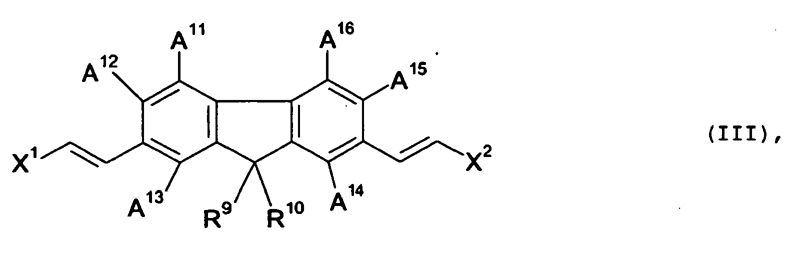
wherein
A¹¹ to A¹⁶ mutually independent mean a substituent selected from a non-metallic atom group,
R⁹, R¹⁰ mutually independent mean a substituent selected from a non-metallic atom group,
X¹, X² mutually independent mean a group of formula (IV) 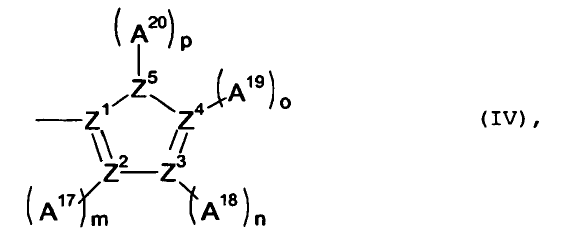 wherein Z¹ to Z⁵ mutually independently mean non-hydrogen, non-metallic atoms,
   A¹⁷ to A²⁰ mutually independent mean a substituent selected from a non-metallic atom group,
   m, n, o mutually independent mean 0 or 1 and
   p means 0, 1 or 2;
   and wherein one or more pairs of the substituents in formulae (III) and/or (IV) can jointly mean the remaining atoms to form a ring;
   with the proviso that if R⁹, R¹⁰ and A¹¹ to A¹⁶ all mean a hydrogen atom, then X¹ and X² do not both mean an unsubstituted 2-thienyl group and do not both mean an unsubstituted 2-benzoxazolyl-group.

The values of m,n,o and p are determined by the possible valencies of the atoms Z¹ to Z⁵. The values of m, n and o calculate as possible valency of Z², Z³ or Z⁴ respectively minus 3 and the value of p calculates as possible valency of Z⁵ minus 2. A possible valency is defined in the context of the present invention as the valency or the valencies an atom Z¹ to Z⁵ can have in a ring of formula (IV). Examples of possible valencies are 4 for a carbon atom, 3 for an uncharged and 4 for a positively charged nitrogen atom, 2 for an oxygen atom and 2 for a sulfur atom.

Preferably Z¹ to Z⁴ mutually independent mean an unsubstituted nitrogen atom, or a carbon atom that is substituted by a substituent selected from a non-metallic atom group,
Z⁵-(A²⁰)ₚ means O, S, CH₂, CHR¹¹, CR¹²R¹³ or NR¹⁴ and
R¹¹ to R¹⁴ mutually independent mean a substituent selected from a non-metallic atom group.

The non-metallic atom group according to the present invention preferably consists of a hydrogen atom or alkyl, alkenyl, alkynyl, aryl, heterocyclyl, hydroxy, carboxy, carbalkoxy, halogeno, alkoxy, aryloxy, heterocyclyloxy, alkylthio, arylthio, heterocyclylthio, alkylseleno, arylseleno, heterocyclylseleno, acyl, acyloxy, alkylsulfonyl, aminosulfonyl, acylamino, cyano, nitro, amino or mercapto groups, wherein heterocycle means a saturated, unsaturated or aromatic heterocycle and acyl means the remaining residue of an aliphatic, olefinic or aromatic carbon, carbaminic, sulfonic, amidosulfonic or phosphonic acid.

In a preferred embodiment of the present invention the non-metallic atom group consists of a hydrogen atom or alkyl, alkenyl, aryl, heterocyclyl, hydroxy, carboxy, carbalkoxy, halogeno, alkoxy, aryloxy, heterocyclyloxy, alkylthio, arylthio, heterocyclylthio, acyl, acyloxy, acylamino, cyano, nitro, amino, or mercapto groups, wherein heterocycle has the same meaning as given above and acyl means the remaining residue of an aliphatic, olefinic or aromatic carbon, sulfonic, amidosulfonic or phosphonic acid.

Alkyl, alkylene, alkenyl, alkenylene, alkynyl and alkynylene groups according to the present invention can be linear (straight chain), branched or cyclic.

The alkyl, alkylene, alkenyl, alkenylene, alkynyl, alkynylene, aryl, heterocyclyl, alkoxy and alkylthio groups of the present invention can be optionally substituted by a substituent selected from the non-metallic atom group of the present invention and the substituents can be selected to adjust the solubility of the divinylfluorene compound and preferably may be halogeno, alkoxy, alkylthio, carbalkoxy, acyloxy or hydroxy.

Said one or more pairs of substituents that jointly can mean the remaining atoms to form a ring preferably are selected from A¹ with R³ ; R³ with R⁴ ; R⁴ with R⁵ ; R⁵ with A²; A⁷ with R¹ or R²; R¹ with R²; A⁸ with R¹ or R²; A³ with R⁶; R⁶ with R⁷; R⁷ with R⁸; R⁸ with A⁴; A⁹ with A¹⁰; A⁵ with A⁶; A¹³ with R⁹ or R¹⁰; R⁹ with R¹⁰; A¹⁴ with R⁹ or R¹⁰; A¹⁵ with A¹⁶; A¹¹ with A¹²; A¹⁷ with A¹⁸; A¹⁸ with A¹⁹; and A¹⁹ with A²⁰.

In a particular preferred embodiment of the present invention, said one or more pairs of substituents that jointly can mean the remaining atoms to form a ring are selected from R³ with R⁴; R⁴ with R⁵; R¹ with R²; R⁶ with R⁷; R⁷ with R⁸; R⁹ with R¹⁰; A¹⁷ with A¹⁸; A¹⁸ with A¹⁹; and A¹⁹ with A²⁰.

A ring according to the present invention means a carbo- or heterocyle, that can be substituted by substituents selected from e.g. the non-metallic atom group of the present invention, that can be saturated, unsaturated or aromatic and that itself can be substituted by further rings. Preferably the ring is a 5 to 8 membered ring, and in particular a 5 or 6 membered ring.

In a further preferred embodiment of the present invention the divinylfluorene compound has a structure according to formula (II), wherein at least one of the substituents R¹ to R⁸ is different from a hydrogen atom or of formula (III), wherein at least one of the substituents R⁹ or R¹⁰ is different from a hydrogen atom.

In another preferred embodiment of the present invention the divinylfluorene compound has a structure according to one of formulae (II) or (III), wherein
A¹ to A¹⁶ mean hydrogen and/or
R³ to R⁸ mutually independent mean a substituent selected from hydrogen or alkyl and/or
R¹, R², R⁹, R¹⁰ mutually independent mean straight chain or branched alkyl preferably having 1 to 20 carbon atoms and particularly preferred having 1 to 10 carbon atoms.

If the divinylfluorene compound of the present invention is used as a sensitizer, further advantages with respect to the sensitivity can be achieved with compounds of the following general formula (III-A): 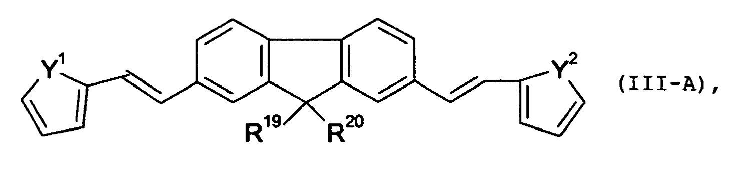 wherein
Y¹, Y² mutually independent mean O, S, NH or N-alkyl and
R¹⁹, R²⁰ mutually independent mean an unsubstituted linear or branched alkyl group having 1 to 20 carbon atoms and preferably having 1 to 10 carbon atoms,
and wherein the hetero rings comprising Y¹ and Y² are mutually independent each substituted by three members selected from a non-metallic atom group. Preferably the hetero rings are each substituted by at least one hydrogen atom, in particular by at least two hydrogen atoms and further preferred by three hydrogen atoms.

When used as sensitizers, further advantages with respect to the sensitivity can be achieved with symmetrical divinylfluorenes. A symmetrical divinylfluorene compound according to the present invention means a compound of formula (II), wherein A¹ = A⁴, A² = A³, R³ = R⁸, R⁴ = R⁷, R⁵ = R⁶, A⁵ = A¹⁰, A⁶ = A⁹, A⁷ = A⁸ and R¹ = R²; or a compound of formula (III), wherein A¹¹ = A¹⁶, A¹² = A¹⁵, A¹³ = A¹⁴, R⁹ = R¹⁰ and X¹ = X².

Divinylfluorene compounds of structure (II) are preferred over those of structure (III).

The following structures are examples of preferred divinylfluorene compounds of the present invention:

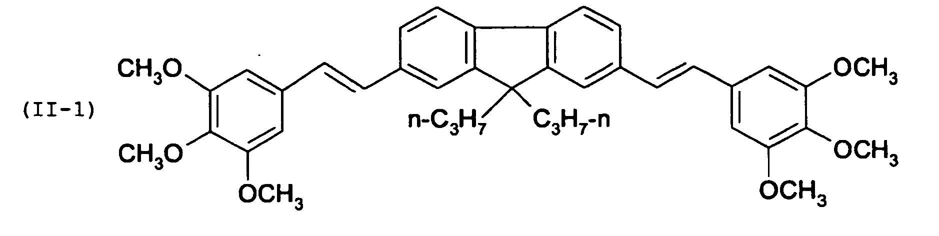

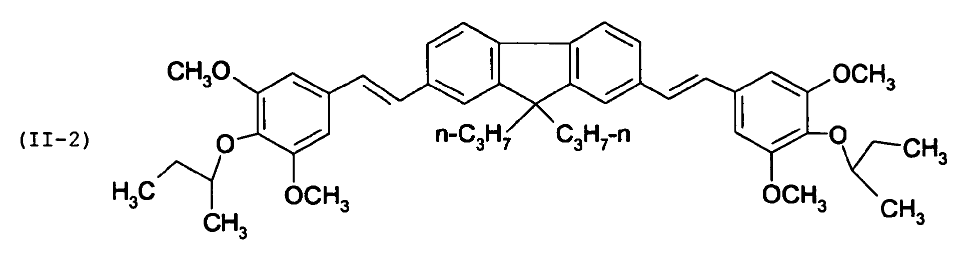

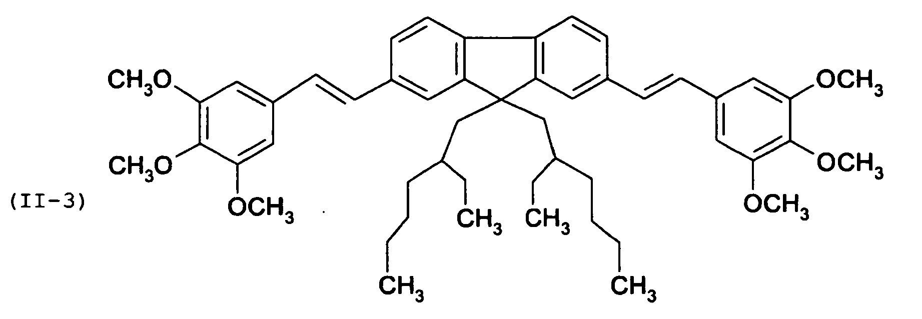

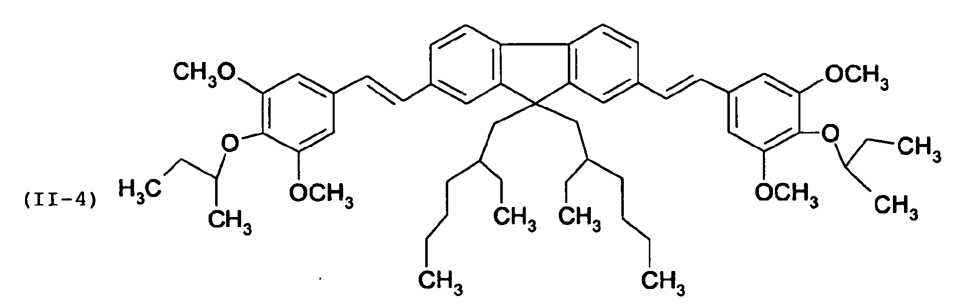

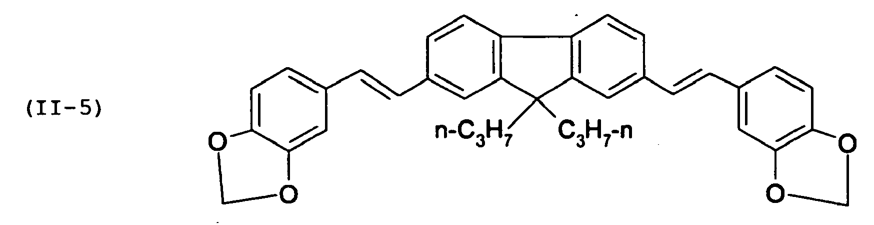

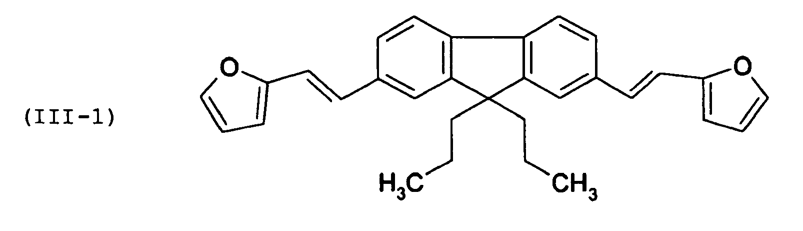

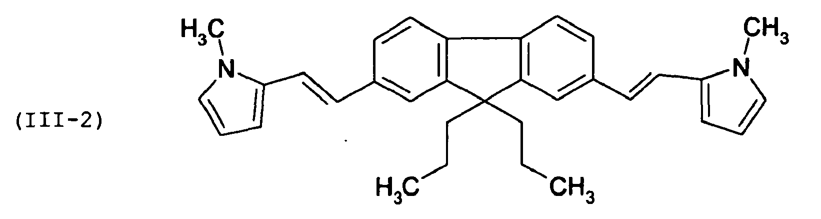

The divinylfluorene compound of the present invention can be used as a single compound or as a mixture of compounds of formulae (II) and/or (III). The overall amount of these compounds ranges from 0.1 to 10 % by weight, preferably 0.5 to 8 % by weight with respect to the total weight of the non-volatile compounds in the composition. The divinylfluorene compound of the present invention can also be combined with known divinylfluorenes and/or with known compounds having the same function, e.g. known sensitizers, optical brighteners or electroluminescent materials.

The divinylfluorene compounds useful for the present invention, in particular when used as sensitizers, preferably have a good solubility in common solvents. It has been found that compounds having a solubility of 0.5 g, in particular 1.5 g sensitzer per 100 mL methylethylketone or more are particularly advantageous as sensitizers with respect to pinhole defects as well as sensitivity.

The compounds of formulae (II) and/or (III) are preferably used as sensitizers in composition that are photopolymerizable upon absorption of light in the wavelength range from 350 to 430 nm, preferably from 380 to 430 nm and in particular from 390 to 420 nm.

The divinylfluorene compounds of the present invention can be synthesized by known methods as disclosed above, but preferably they are synthesized by a new synthetic route that surprisingly has been found and that allows to synthesize a broad range of divinyl fluorene compounds by only four steps that all give high yields and only require readily available reactants.

This new synthetic method of the present invention is characterized in that in the first step the 9-position of a 2,7,9-unsubstituted fluorene compound (substituted by hydrogen atoms at positions 2, 7 and 9) is dialkylated using a strong base and an alkyl halide, in step 2 the 9,9-dialkylated fluorene is 2,7-bis-halomethylated, this intermediate is then reacted in step 3 with trialkylphosphite to 2,7-bis(dialkylphosphonatomethyl)-9,9-dialkylfluorene, which gives in step 4 on reaction with an aromatic aldehyde 2,7-divinylfluorene compounds, in particular those of formulae (II) and (III), in high yield.

Step 1 preferably is done in polar aprotic solvents like dimethylformamide (DMF), dimethylsulfoxide (DMSO) or an ether like tetrahydrofurane (THF). The fluorene is first deprotonated, in particular using strong bases like sodium hydride, n-butyl lithium (LiButyl-n) or tert-butyl lithium (LiButyl-t), and then the alkyl halide, preferably a primary or secondary alkyl bromide or chloride is carfully added.

Step 2 preferably is done with paraformaldehyde and hydrobromic acid.

Step 4 is preferably done with a base such as sodium hydride (NaH), sodium ethoxylate (NaOEthyl), sodium methoxylate (NaOMethyl), lithium ethoxylate (LiOEthyl), lithium methoxylate (LiOMethyl), potassium tert-butyloxylate (KOButyl-t), sodium tert-butyloxylate (NaOButyl-t), lithium hydroxide (LiOH), potassium hydroxide (KOH), di-sodium carbonate (Na₂CO₃) or di-potassium carbonate (K₂CO₃) in a polar solvent such as DMF, ethanol, DMSO, sulpholane or an ether like THF.

The following example synthesis of compounds (II-1), (II-2) and (II-5) are good starting points for the conditions and reagents useful for the synthetic method of the present invention, but the present invention is not restricted to this example. On the contrary a person skilled in the art can easily adapt the conditions such as reaction temperatures and times as well as the solvents and can use known functionally equivalent reagents to yield with only 4 steps a wide variety of divinylfluorenes as very pure products without the necessity of difficult purifying steps and only using readily available reagents.

### Example synthesis of divinylfluorene (II-1)

### Step 1: 9,9-Dipropylfluorene (2)

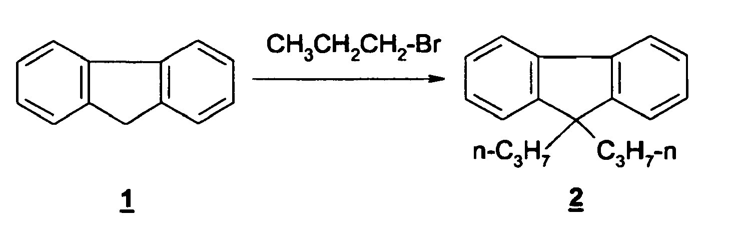

To a solution of fluorene (**1**) (41.5g) in dimethylformamide (300 mL) at 20°C was added sodium hydride (24.7g) in portions. The red solution was stirred for 2 hours at 35°C until no more gas was evolved. To this solution was added 1-bromopropane (62.7g) drop-wise over 1 hour at 5°C and then the mixture was stirred for 1 hour at 40°C.

The suspension was poured into ice-water (1.5L) and the resultant oil was dissolved in methylene chloride (0.5L). The organic phase was washed with water, dried over magnesium sulfate and the solvent was removed under reduced pressure. The residue was purified by vacuum distillation (122-125°C/0.5 mmHg). After cooling, **2** was obtained as a crystalline product (47.0g, 74%).

### Step 2: 2,7-Bis(bromomethyl)-9,9-dipropylfluorene (3)

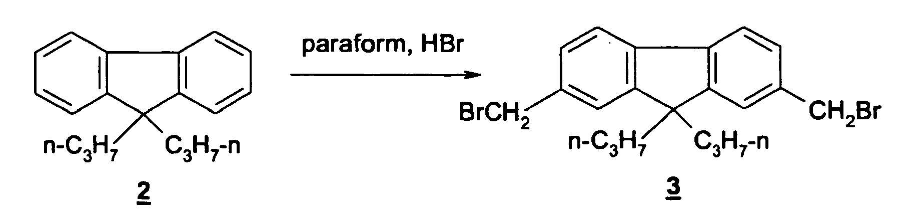

To a solution of 9,9-dipropylfluorene **2** (25.0g) in acetic acid (50mL) was added paraformaldehyde (paraform) (18.0g) at 15°C. To the mixture was then added a solution of hydrogen bromide in acetic acid (250 mL, 30% w/w) over 0.5 hours, and the solution was stirred at 60°C for 5 hours. The reaction mixture was poured into ice-water (1.0L) and stirred for 0.5 hours. The precipitate was filtered off and purified by stirring in acetonitrile (200 mL) at 40°C, filtering and drying to give **3** as a yellow powder (33.g, 77%).

### Step 3: 2,7-Bis(diethylphosphonatomethyl)-9,9-dipropylfluorene (4)

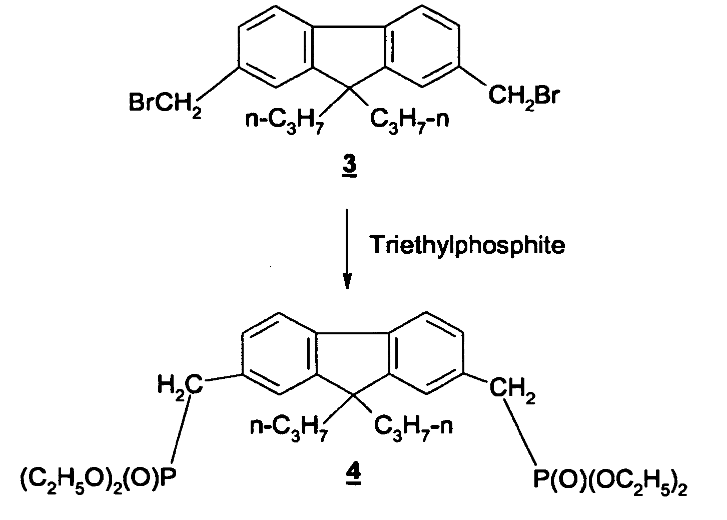

A mixture of 2,7-Bis(bromomethyl)-9,9-dipropylfluorene (**3**) (33.0g) and triethylphosphite (40 mL) was stirred for 3 hours at 150°C. Excess triethylphosphite was removed at reduced pressure at 100°C and the resultant oil crystallised from hexane. After drying, **4** was obtained as a white powder (31.5g, 67.5%).

### Step 4: divinylfluorene (II-1)

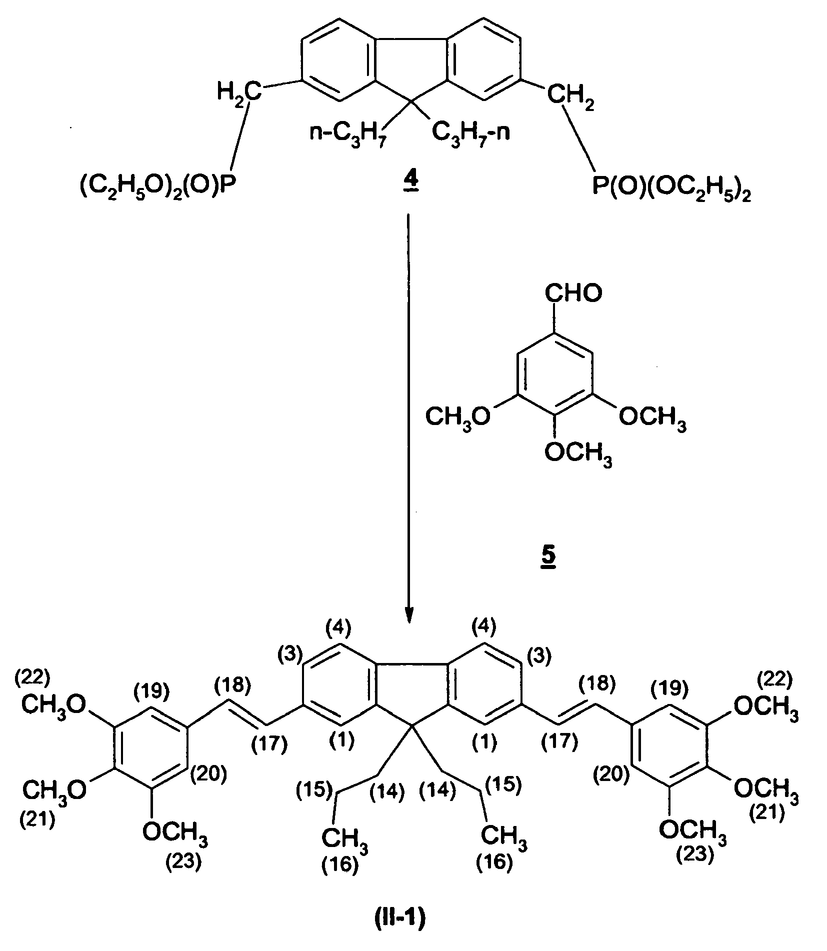

A suspension of 2,7-Bis(diethylphosphonatomethyl)-9,9-dipropylfluorene (**4**) (2.15g, 4.0 mmol), 3,4,5-trimethoxybenzaldehyde (**5**) (1.76g, 9.0 mmol), and potassium hydroxide (0.75g, 13 mmol) in THF (20 mL) and DMSO (1 mL) was heated at 70°C for 4 hours. The suspension was diluted with ethanol (50mL) and the solvent removed under vacuum. The product was purified by refluxing in methanol followed by filtration. After drying, (**II-1**) was obtained as a pale yellow powder (1.90 g, 76 %). The UV/VIS spectrum of (II-1) in methyl ethyl ketone (MEK) has two maxima at 380 nm and at 399 nm with a molar extinction coefficient of 89724 Mol⁻¹cm⁻¹ and 72301 Mol⁻¹cm⁻¹ respectively. Under UV irradiation in MEK, (II-1) shows a strong blue fluorescence with fluorescence peaks at 417 nm and 439 nm. The fluorescence quantum yield of (II-1) relative to the anthracene standard is 0.65 (in MEK solvent, not degassed). The ¹H-NMR data in CDCl₃ are summarized in table 1; in the table s means singlet, d means doublet, dd means double doublet, m means multiplet and J means the coupling constant.

**Table 1 ¹H-NMR data of (II-1)**

| Hydrogen position | Chemical shift / ppm | Coupling (Coupling constant) |
|---|---|---|
| (3) | 7.49 | dd (J = 8Hz, 2Hz) |
| (4) | 7.67 | d (J = 8Hz) |
| (1) | 7.48 | d (J = 2Hz) |
| (14) | 2.01 | m |
| (15) | 0.71 | m |
| (16) | 0.71 | m |
| (17) | 7.10 | d (J = 16Hz) |
| (18) | 7.10 | d (J = 16Hz) |
| (19), (20) | 6.79 | s |
| (21) | 3.89 | s |
| (22), (23) | 3.93 | s |

### Example synthesis of divinylfluorene (II-2)

Steps 1 to 3 were run in the same way as described for the synthesis of (II-1).

### Step 4: divinylfluorene (II-2)

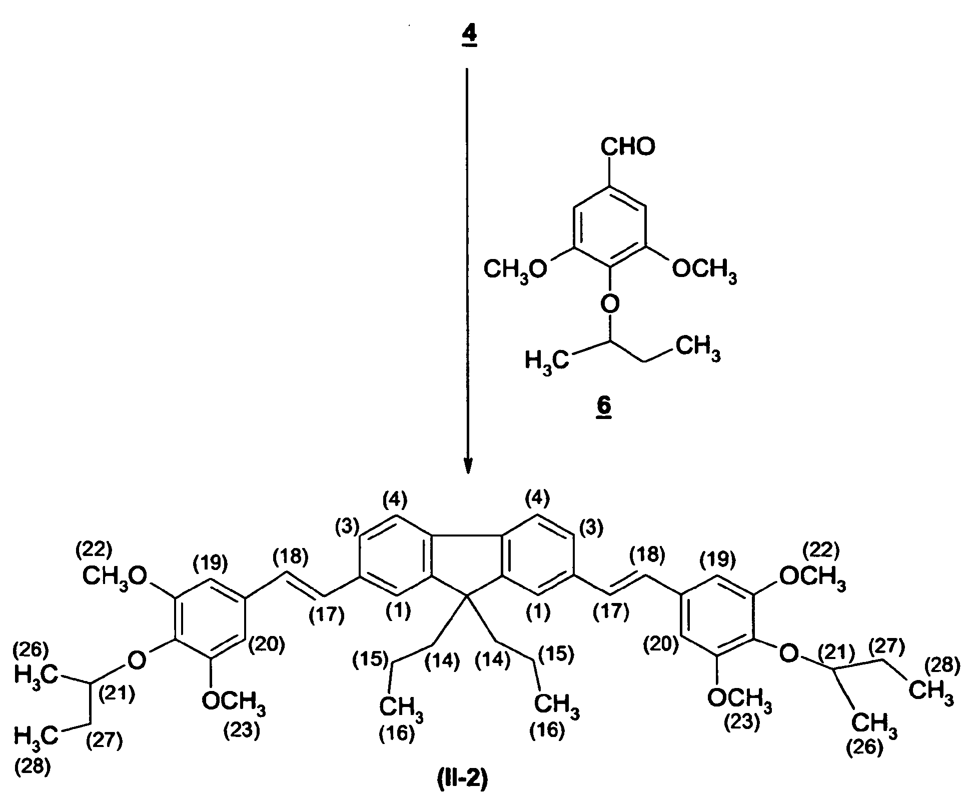

To a solution of 2,7-bis (diethylphosphonatomethyl)-9,9-dipropylfluorene (**4**) (26.8g) and 3,5-dimethoxy-4-(1-methylpropoxy)-benzaldehyde (**6**) (26.2g) in tetrahydrofuran (200 mL) was added potassium hydroxide (8.4g) and dimethylsulfoxide (5.0mL). The suspension was stirred at 70°C for 4 hours and then isopropanol (150mL) was added. The solvent of the supernatant solution was removed at reduced pressure and the resultant oil was stirred in methanol (200 mL). The precipitate was filtered off and purified by stirring in boiling ethanol (200 mL) two times. After drying, (**II-2**) was obtained as a pale yellow powder (25.8 g, 72 %). The UV/VIS spectrum of (II-2) in methyl ethyl ketone (MEK) has two maxima at 382 nm and at 402 nm with a molar extinction coefficient of 93092 Mol⁻¹cm⁻¹ and 75139 Mol⁻¹cm⁻¹ respectively. Under UV irradiation in MEK, (II-2) shows a strong blue fluorescence with fluorescence peaks at 419 nm and 444 nm. The fluorescence quantum yield of (II-2) relative to the anthracene standard is 0.66 (in MEK solvent, not degassed). The ¹H-NMR data in CDCl₃ are summarized in table 2.

**Table 2 ¹H-NMR data of (II-2)**

| Hydrogen position | Chemical shift / ppm | Coupling (Coupling constant) |
|---|---|---|
| (3) | 7.49 | dd (J = 8Hz, 2Hz) |
| (4) | 7.65 | d (J = 8Hz) |
| (1) | 7.48 | d (J = 2Hz) |
| (14) | 2.01 | m |
| (15) | 0.71 | m |
| (16) | 0.71 | m |
| (17) | 7.10 | d (J = 16Hz) |
| (18) | 7.10 | d (J = 16Hz) |
| (19), (20) | 6.79 | s |
| (21) | 4.20 | m |
| (22), (23) | 3.90 | s |
| (26) | 1.25 | d (J = 6Hz) |
| (27) | 1.80, 1.61 | m |
| (28) | 1.00 | t (J = 7Hz) |

### Example synthesis of divinylfluorene (II-5)

Steps 1 to 3 were run in the same way as described for the synthesis of (II-1).

### Step 4: divinylfluorene (II-5)

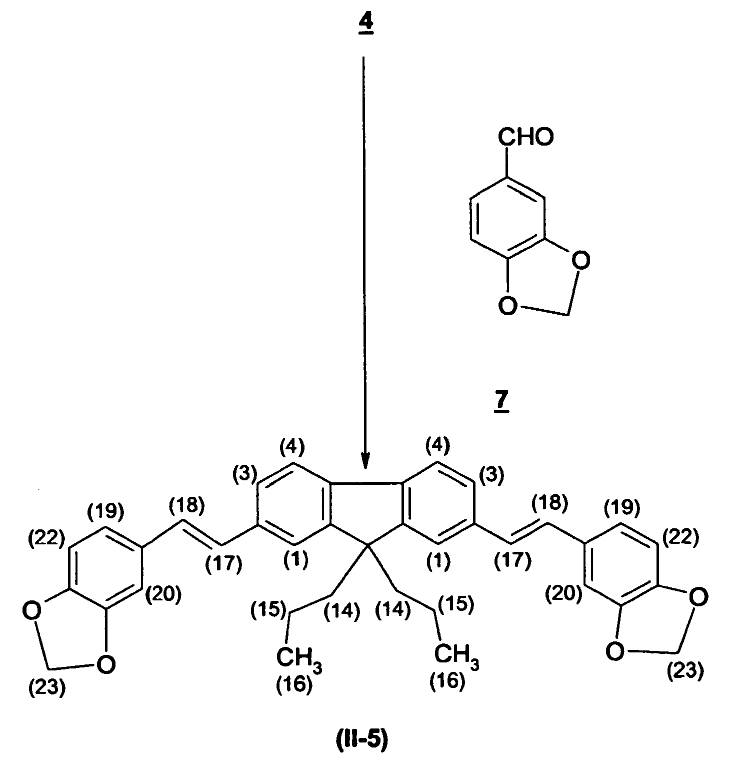

A suspension of 2,7-bis(diethylphosphonatomethyl)-9,9-dipropylfluorene (**4**) (2.70g, 5 mmol), 3,4-methylenedioxybenzaldehyde (**7**) (1.60 g, 11 mmol) and potassium hydroxide (0.85 g, 15 mmol) in THF (30 mL) and DMSO (1 mL) was heated at 70°C for 7 hours. The suspension was diluted with ethanol (50 mL) and the solvent removed under vacuum. The product was purified by refluxing in methanol followed by filtration. After drying, (**II-5**) was obtained as a pale yellow powder (1.95 g, 72%). The UV/VIS spectrum of (II-5) in methyl ethyl ketone (MEK) has two maxima at 382 nm and at 401 nm with a molar extinction coefficient of 79099 Mol⁻¹cm⁻¹ and 63911 Mol⁻¹cm⁻¹ respectively. Under UV irradiation (II-5) shows a strong blue fluorescence. The ¹H-NMR data in CDCl₃ are summarized in table 3.

**Table 3 ¹H-NMR data of (II-5)**

| Hydrogen position | Chemical shift / ppm | Coupling (Coupling constant) |
|---|---|---|
| (23) | 6.00 | s |
| (22) | 6.82 | d (J = 8Hz) |
| (19) | 6.99 | d (J = 8Hz) |
| (20) | 7.11 | s |
| (18) | 7.03 | d (J = 16.4Hz) |
| (17) | 7.10 | d (J = 16.4Hz) |
| (1) | 7.46 | d (2Hz) |
| (3) | 7.46 | d (J = 8Hz, 2Hz) |
| (4) | 7.64 | d (J = 8Hz) |
| (14) | 2.00 | m |
| (15) | 0.70 | m |
| (16) | 0.70 | m |

When used as sensitizers for photopolymerizable compositions, such compositions preferably are photopolymerizable upon absorption of light in the wavelength range from 300 to 450 nm and comprise a binder, a polymerizable compound, a photoinitiator and a sensitizer, wherein the sensitizer is a divinyl fluorene compound of the present invention. The known photopolymerization initiators can be used in combination with the divinylfluorene compounds of the present invention. Suitable classes of photopolymerization initiators include aromatic ketones, aromatic onium salts, organic peroxides, thio compounds, hexaarylbisimidazole compounds, ketooxime ester compounds, borate compounds, azinium compounds, metallocene compounds, active ester compounds and compounds having a carbon-halogen bond. Many specific examples of such photoinitiators can be found in EP-A 1091247.

Good results, in particular high sensitivity, can be obtained by the combination of a divinyl fluorene sensitizer according to the present invention and a hexaarylbisimidazole (HABI, dimer of triaryl-imidazole) as photoinitiator.

A procedure for the preparation of HABIs is described in DE 1470 154 and their use in photopolymerizable compositions is documented in EP 24 629, EP 107 792, US 4 410 621, EP 215 453 and DE 3 211 312. Preferred derivatives are e. g. 2,4,5,2',4',5'-hexaphenyl-bisimidazole, 2,2'-bis(2-chlorophenyl)-4,5,4',5'-tetraphenylbisimidazole, 2,2'-bis(2-bromophenyl)-4,5,4',5'-tetraphenylbisimidazole, 2,2'-bis(2,4-dichlorophenyl)-4,5,4',5'-tetraphenylbisimidazole, 2,2'-bis(2-chlorophenyl)-4,5,4',5'-tetrakis(3-methoxyphenyl)bisimidazole, 2,2'-bis(2-chlorophenyl)-4,5,4',5'-tetrakis(3,4,5-trimethoxyphenyl)-bisimidazole, 2,5,2',5'-tetrakis(2-chlorophenyl)-4,4'-bis(3,4-dimethoxyphenyl)bisimidazole, 2,2'-bis(2,6-dichlorophenyl)-4,5,4',5'-tetraphenylbisimidazole, 2,2'-bis(2-nitrophenyl)-4,5,4',5'-tetraphenylbisimidazole, 2,2'-di-o-tolyl-4,5,4',5'-tetraphenylbisimidazole, 2,2'-bis(2-ethoxyphenyl)-4,5,4',5'-tetraphenylbisimidazole and 2,2'-bis(2,6-difluorophenyl)-4,5,4',5'-tetraphenylbisimidazole. The amount of the HABI photoinitiator typically ranges from 0.01 to 30 % by weight, preferably from 0.5 to 20 % by weight, relative to the total weight of the non volatile components of the photopolymerizable composition.

The binder can be selected from a wide series of organic polymers. Compositions of different binders can also be used. Useful binders include for example chlorinated polyalkylenes in particular chlorinated polyethylene and chlorinated polypropylene; poly(methacrylic acid) alkyl esters or alkenyl esters in particular poly(methyl (meth)acrylate), poly(ethyl (meth)acrylate), poly(butyl (meth)acrylate), poly(isobutyl (meth)acrylate), poly(hexyl (meth)acrylate), poly((2-ethylhexyl) (meth)acrylate) and poly(alkyl (meth)acrylate); copolymers of (meth)acrylic acid alkyl esters or alkenyl esters with other copolymerizable monomers, in particular with (meth)acrylonitrile, vinyl chloride, vinylidene chloride, styrene and/or butadiene; poly(vinyl chloride) (PVC); vinylchloride/(meth)acrylonitrile copolymers; poly(vinylidene chloride) (PVDC); vinylidene chloride/(meth)acrylonitrile copolymers; poly(vinyl acetate); poly(vinyl alcohol); poly (meth)acrylonitrile; (meth)acrylonitrile/styrene copolymers; (meth)acrylamide/alkyl (meth)acrylate copolymers; (meth)acrylonitrile/butadiene/styrene (ABS) terpolymers; polystyrene; poly(α-methylstyrene); polyamides; polyurethanes; polyesters; cellulose or cellulose compounds like methyl cellulose, ethyl cellulose, acetyl cellulose, hydroxy-(C₁₋₄₋alkyl)cellulose, carboxymethyl cellulose; poly(vinyl formal) and poly(vinyl butyral). Particularly suitable are binders that are insoluble in water, but on the other hand are soluble or at least swellable in aqueous-alkaline solutions. Further effective binders are polymers that are soluble in common organic coating solvents.

Particular suitable for the purpose of the present invention are binders containing carboxyl groups, in particular polymers or copolymers containing monomeric units of α,β-unsaturated carboxylic acids and/or monomeric units of α,β-unsaturated dicarboxylic acids, preferably acrylic acid, methacrylic acid, crotonic acid, vinylacetic acid, maleic acid or itaconic acid. By the term "copolymers" are to be understood in the context of the present invention polymers containing units of at least 2 different monomers, thus also terpolymers and higher mixed polymers. Particular useful examples of copolymers are those containing units of (meth)acrylic acid and units of alkyl (meth)acrylates, allyl (meth)acrylates and/or (meth)acrylonitrile as well as copolymers containing units of crotonic acid and units of alkyl (meth)acrylates and/or (meth)acrylonitrile and vinylacetic acid/alkyl (meth)acrylate copolymers. Also suitable are copolymers containing units of maleic anhydride or maleic acid monoalkyl esters. Among those are, for example, copolymers containing units of maleic anhydride and styrene, unsaturated ethers or esters or unsaturated aliphatic hydrocarbons and the esterification products obtained from such copolymers. Further suitable binders are products obtainable from the conversion of hydroxyl-containing polymers with intramolecular dicarboxylic anhydrides. Further useful binders are polymers in which groups with acid hydrogen atoms are present, some or all of which are converted with activated isocyanates. Examples of these polymers are products obtained by conversion of hydroxyl-containing polymers with aliphatic or aromatic sulfonyl isocyanates or phosphinic acid isocyanates. Also suitable are polymers with aliphatic or aromatic hydroxyl groups, for example copolymers containing units of hydroxyalkyl (meth)acrylates, allyl alcohol, hydroxystyrene or vinyl alcohol, as well as epoxy resins, provided they carry a sufficient number of free OH groups.

The organic polymers used as binders have a typical mean molecular weight M_{w} between 600 and 200 000, preferably between 1 000 and 100 000. Preference is further given to polymers having an acid number between 10 to 250, preferably 20 to 200, or a hydroxyl number between 50 and 750, preferably between 100 and 500. The amount of binder(s) generally ranges from 10 to 90 % by weight, preferably 20 to 80 % by weight, relative to the total weight of the non-volatile components of the composition.

The polymerizable compound can be selected from a wide series of photo-oxidizable compounds. Suitable compounds contain primary, secondary and in particular tertiary amino groups. Radically polymerizable compounds containing at least one urethane and/or urea group and/or a tertiary amino group are particularly preferred. By the term "urea group" has to be understood in the context of the present invention a group of the formula >N-CO-N<, wherein the valences on the nitrogen atoms are saturated by hydrogen atoms and hydrocarbon radicals (with the proviso that not more than one valence on either of the two nitrogen atoms is saturated by one hydrogen atom). However, it is also possible for one valence on one nitrogen atom to be bonded to a carbamoyl (-CO-NH-) group, producing a biuret structure.

Also suitable are compounds containing a photo-oxidizable amino, urea or thio group, which may be also be a constituent of a heterocyclic ring. Compounds containing photo-oxidizable enol groups can also be used. Specific examples of photo-oxidizable groups are triethanolamino, triphenylamino, thiourea, imidazole, oxazole, thiazole, acetylacetonyl, N-phenylglycine and ascorbic acid groups. Particularly suitable compounds are monomers containing photo-oxidizable groups corresponding to the following formula (XVIII):

R₍ₘ₋ₙ₎Q[(-CH₂-CR¹R²-O)ₐ-CO-NH-(X¹-NH-CO-O)_{b}-X²-(O-CO-CR³=CH₂)_{c}]ₙ (XVIII)

wherein
- R: represents an alkyl group having 2 to 8 carbon atoms ((C₂-C₈) alkyl group), a (C₂-C₈) hydroxyalkyl group or a (C₆-C₁₄) aryl group,
- Q: represents -S-, 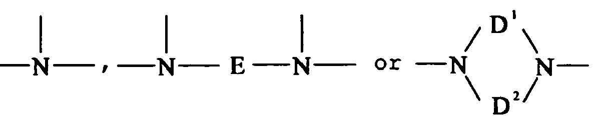 wherein
E represents a divalent saturated hydrocarbon group of 2 to 12 carbon atoms, a divalent 5- to 7-membered, saturated iso- or heterocyclic group, which may contain up to 2 nitrogen, oxygen and/or sulfur atoms in the ring, a divalent aromatic mono- or bicyclic isocyclic group of 6 to 12 carbon atoms or a divalent 5- or 6-membered aromatic heterocyclic group; and
D¹ and D² independently represent a saturated hydrocarbon group of 1 to 5 carbon atoms,
- R¹: and R² independently represent a hydrogen atom, an alkyl or alkoxyalkyl group,
- R³: represents a hydrogen atom, a methyl or ethyl group,
- X¹: represents a straight-chained or branched saturated hydrocarbon group of 1 to 12 carbon atoms,
- X²: represents a (c+1)-valent hydrocarbon group in which up to 5 methylene groups may have been replaced by oxygen atoms,
- a: is an integer from 0 to 4,
- b: is 0 or 1,
- c: is an integer from 1 to 3,
- m: is an integer from 2 to 4 and
- n: is an integer from 1 to m.

Compounds of this nature and processes for their preparation are described in EP 287 818. If a compound of general formula (XVIII) contains several radicals R or several radicals according to the structure indicated between square brackets, i. e. if (n-m) > 1 and n>l, these radicals can be identical or different from one another. Compounds according to formula (XVIII) wherein n = m are particularly preferred. In this case, all radicals contain polymerizable groups. Preferably, the index a is 1; if several radicals are present, a cannot be 0 in more than one radical. If R is an alkyl or hydroxyalkyl group, R generally contains 2 to 6, particularly 2 to 4 carbon atoms. Aryl radicals R are in general mononuclear or binuclear, preferably however mononuclear, and may be substituted with (C₁-C₅) alkyl or (C₁-C₅) alkoxy groups. If R¹ and R² are alkyl or alkoxy groups, they preferably contain 1 to 5 carbon atoms. R³ is preferably a hydrogen atom or a methyl group. X¹ is preferably a straight-chained or branched aliphatic and/or cycloaliphatic radical of preferably 4 to 10 carbon atoms. In a preferred embodiment, X² contains 2 to 15 carbon atoms and is in particular a saturated, straight-chained or branched aliphatic and/or cycloaliphatic radical containing this amount of carbon atoms. Up to 5 methylene groups in these radicals may have been replaced by oxygen atoms; in the case of X² being composed of pure carbon chains, the radical generally has 2 to 12 carbon atoms, preferably 2 to 6 carbon atoms. X² can also be a cycloaliphatic group of 5 to 10 carbon atoms, in particular a cyclohexane diyl group. The saturated heterocyclic ring formed by D¹, D² and both nitrogen atoms generally has 5 to 10 ring members in particular 6 ring members. In the latter case the heterocyclic ring is preferably a piperazine and the radical derived therefrom a piperazine-1,4-diyl radical. In a preferred embodiment, radical E is an alkane diyl group which normally contains about 2 to 6 carbon atoms. Preferably the divalent 5- to 7-membered, saturated, isocyclic group E is a cyclohexane diyl group, in particular a cyclohexane-1,4-diyl group. The divalent, isocyclic, aromatic group E is preferably an ortho-, meta- or para-phenylene group. The divalent 5-or 6-membered aromatic heterocyclic group E, finally, contains preferably nitrogen and/or sulphur atoms in the heterocyclic ring. c is preferably 1, i. e. each radical in the square bracket generally contains only one polymerizable group, in particular only one (meth)acryloyloxy-group.

The compounds of formula (XVIII) wherein b = 1, which accordingly contain two urethane groups in each of the radicals indicated in the square brackets, can be produced in a known way by conversion of acrylic esters or alkacrylic esters which contain free hydroxyl groups with equimolar amounts of diisocyanates. Excess isocyanate groups are then, for example, reacted with tris(hydroxyalkyl)amines, N,N'-bis(hydroxyalkyl) piperazines or N,N,N',N'-tetrakis(hydroxyalkyl)alkylenediamines, in each of which individual hydroxyalkyl groups may have been replaced by alkyl or aryl groups R. If a = 0, the result is a urea grouping. Examples of the hydroxyalkylamine starting materials are diethanolamine, triethanolamine, tris(2-hydroxypropyl)amine, tris(2-hydroxybutyl)amine and alkyl-bis-hydroxyalkylamines. Examples of suitable diisocyanates are hexamethylene diisocyanate, 2,2,4-trimethylhexamethylene diisocyanate, 1,4-cyclohexylene diisocyanate (= 1,4-diisocyanatocyclohexane) and 1,1,3-trimethyl-3-isocyanatomethyl-5-isocyanatocyclohexane. The hydroxy-containing esters used are preferably hydroxyethyl (meth)acrylate, hydroxypropyl (meth)acrylate and hydroxyisopropyl (meth)acrylate.

The polymerizable compounds of formula (XVIII) wherein b = 0 are prepared converting the above-described hydroxyalkylamino compounds with isocyanate-containing acrylic or alkacrylic esters. A preferred isocyanate-containing ester is isocyanoto-ethyl (meth)acrylate.

Further polymerizable compounds comprising photooxidisable groups suitable for the purpose of the invention are compounds according to the following formula (XIX):

R₍ₘ₋ₙ₎Q [(-CH₂-R¹R²-O)ₐ- (CH₂-CH[CH₂-O-CO-CR³=CH₂]-O)_{b}-H] ₙ (XIX)

wherein a' and b' independently represent integers from 1 to 4 and Q, R¹, R², R³, n and m have the same meaning as above and Q can also be a group of the formula >N-E'-N< wherein the radical E' corresponds to the following formula (XX):

-CH₂-CH (OH) -CH₂-[O-(p)C₆H₄-C(CH₃)₂- (p)C₆H₄-CH₂-CH(OH)-CH₂-]_{c} (XX)

wherein c has the same meaning as in formula (XVIII) and (p)C₆H₄ represents para-phenylene.

The compounds of formula (XIX) are prepared analogously to those of formula (XVIII), except that the conversion products of hydroxyalkyl acrylates or alkacrylates and diisocyanates are replaced by the corresponding acrylic and alkacrylic glycide esters. Compounds of formula (XX) and processes to their preparation are disclosed in EP 316 706.

Further useful polymerizable compounds containing photooxidisable groups are acrylic and alkacrylic esters of the following formula (XXI) :

Q' [(-X^{1'}-CH₂-O)ₐ-CO-NH(-X¹-NH-CO-O)_{b}-X²-O-CO-CR³=CH₂]ₙ (XXI)

wherein
Q' represents 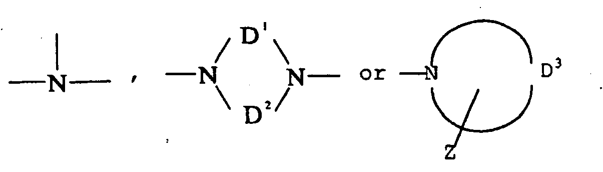 wherein D¹ and D² independently represent a saturated hydrocarbon group of 1 to 5 carbon atoms and D³ represents a saturated hydrocarbon group of 4 to 8 carbon atoms, which together with the nitrogen atom forms a 5- or 6-membered heterocyclic ring;
X^{1'} represents -C₁H₂₁- or 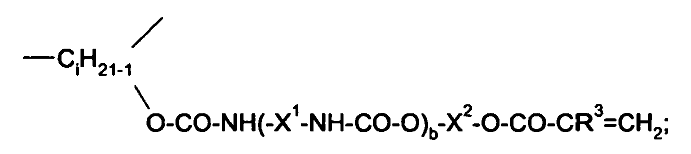 Z represents a hydrogen atom or a radical of the following formula:

-CₖH₂ₖ-O-CO-NH(-X¹-NH-CO-O)_{b}-X²-O-CO-CR³=CH₂ ;

i,k independently represent integers from 1 to 12;
n' represents an integer from 1 to 3; and
a is 0 or 1; provided that a is 0 in at least one of the radicals bonded to Q;
X¹, R³, a and b have the same meaning as given in the above formula (VIII); and
X² represents a divalent hydrocarbon group in which up to 5 methylene groups may be replaced by oxygen atoms.

In formula (XXI) index a is preferably 0 or 1 and i preferably represents a number between 2 and 10. Preferred radicals Q are piperazine-1,4-diyl (D¹ = D² = CH₂-CR₂), piperidine-1-yl (D³ = (CH₂)₅, Z = H) and 2-(2-hydroxyethyl)-piperidine-1-yl (D³ = (CH₂)₅, Z = CH₂CH₂OH).

Of the compounds of formula (XXI), those which apart from a urea group contain at least one urethane group are preferred. Here again, by the term "urea group" has to be understood the group of formula >N-CO-N< already mentioned above. Compounds of formula (XXI) and processes for their preparation are disclosed in EP 355 387.

Also suitable polymerizable compounds are reaction products of mono- or diisocyanates with multifunctional alcohols, in which the hydroxy groups are partly or completely esterified with (meth)acrylic acid. Preferred compounds are materials, which are synthesized by the reaction of hydroxyalkyl-(meth)acrylates with diisocyanates. Such compounds are basically known and for instance described in DE 28 22 190 and
DE 20 64 079.

The amount of polymerizable compound comprising photooxidisable groups generally ranges from 5 to 75 % by weight, preferably from 10 to 65 % by weight, relative to the total weight of the non volatile compounds of the photopolymerizable composition.

Moreover, the composition can contain polyfunctional (meth)acrylate or alkyl(meth)acrylate compounds as crosslinking agents. Such compounds contain more than 2, preferably between 3 and 6 (meth)acrylate and/or alkyl(meth)acrylate groups and include in particular (meth)acrylates of saturated aliphatic or alicyclic trivalent or polyvalent alcohols such as trimethylol ethane, trimethylol propane, pentaerythritol or dipentaerythritol.

The total amount of polymerizable compounds generally ranges from about 10 to 90 % by weight, preferably from about 20 to 80 % by weight, relative to the total weight of the non volatile components of the photopolymerizable composition comprising the divinylfluorene compound of the present invention.

The following specific example is also a suitable polymerizable compound :

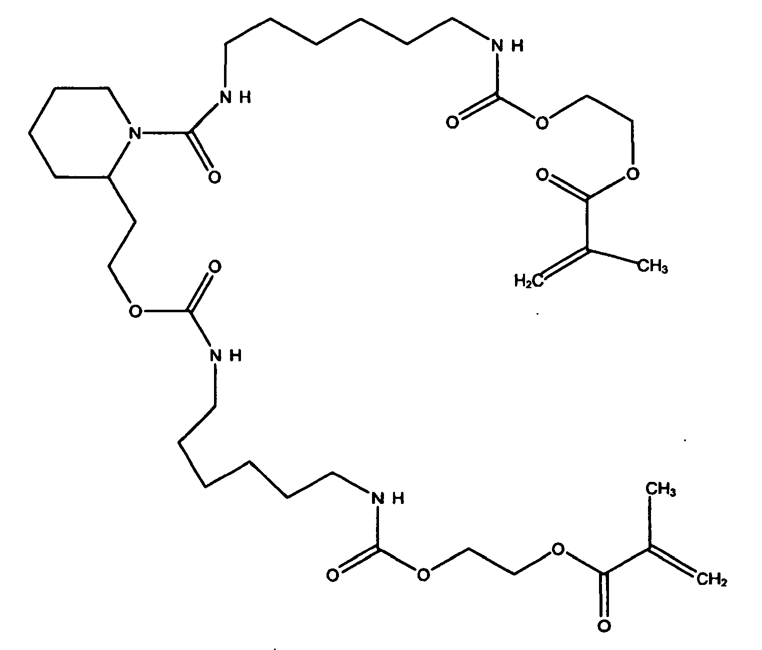

In order to achieve a high sensitivity, it is advantageous to add a radical chain transfer agent as described in EP 107 792 to the photopolymerizable composition in combination with the divinylfluorene compound of the present invention. The preferred chain transfer agents are sulfur containing compounds, especially thiols like e. g. 2-mercaptobenzothiazole, 2-mercaptobenzoxazole or 2-mercapto-benzimidazole. The amount of chain transfer agent generally ranges from 0.01 to 10 % by weight, preferably from 0.1 to 2 % by weight, relative to the total weight of the non volatile components of the photopolymerizable composition.

In order to adjust the photopolymerizable composition comprising the divinyl compound according to the present invention to specific needs, thermal inhibitors or stabilizers for preventing thermal polymerization may be added. Furthermore additional hydrogen donors, dyes, colored or colorless pigments, color formers, indicators and plasticisers may be present. These additives are convieniently selected so that they absorb as little as possible in the actinic range of the imagewise applied radiation.

The photopolymerizable composition comprising the divinyl fluorene according to the present invention is applied to the support by processes which are known per se to the person skilled in the art. In general, the components of the photopolymerizable composition are dissolved or dispersed in an organic solvent or solvent mixture, the solution or dispersion is applied to the intended support by pouring on, spraying on, immersion, roll application or in a similar way, and the solvents are removed during the subsequent drying.

The known supports can be used for the photopolymer printing plate comprising the divinyl fluorene compounds of the present invention, like e. g. foils, tapes or plates made of metal or plastics and in the case of screen-printing also of Perlon gauze. Preferred metals are aluminium, aluminium alloys, steel and zinc, aluminium and aluminium alloys being particularly preferred. Preferred plastics are polyester and cellulose acetates, poly-ethyleneterephthalate (PET) being particularly preferred.

In most cases it is preferred, to treat the surface of the support mechanically and/or chemically and/or electrochemically to optimally adjust the adherence between the support and the photosensitive coating and/or to reduce the reflection of the imagewise exposed radiation on the surface of the support (antihalation).

The most preferred support to be used for the photopolymer printing plates is made of aluminium or an aluminium alloy, its surface is electrochemically roughened, thereafter anodized and optionally treated with a hydrophilizing agent like e. g. poly(vinylphosphonic acid).

Such printing plate precursors preferably have a protective layer (overcoat layer) provided on top of the photopolymerizable layer.

Said protective layer may contain the ingredients known in the art, in particular water soluble polymers like poly(vinyl alcohols) or poly(vinyl pyrrolidone), surface wetting agents, coloring agents, complexants and biocides. Among said complexants, ethoxylated ethylene diamine compounds have been found to be particularly preferred in combination with the divinyl fluorene compounds of the present invention.

Preferably the protective overcoat comprises at least one type of poly(vinyl alcohol), wherein the mean degree of saponification is less than 93 mol-%.

The degree of saponification is related to the production of poly(vinyl alcohols). As the monomer of poly(vinyl alcohol), vinyl alcohol, is nonexistent, only indirect methods are available for the production of poly(vinyl alcohol). The most important manufacturing process for poly(vinyl alcohol) is the polymerization of vinyl esters or ethers, with subsequent saponification or transesterification. The preferred starting material for the poly (vinyl alcohol) used in the context of the present invention is a vinyl alcohol esterified by a mono carboxylic acid and in particular vinyl acetate, but derivatives of vinyl acetate, vinyl esters of di carboxylic acids or vinyl ethers can also be used. The degree of saponification as defined in the context of the present invention is the molar degree of hydrolysis irrespective of the process used for the hydrolysis. Pure poly (vinyl alcohol) has e. g. a degree of saponification of 100 mol-%, but commercial products often have a degree of saponification of 98 mol-%. The poly(vinyl alcohols) as preferably used in the context of the present invention contain mainly 1,3-diol units, but may also contain small amounts of 1,2-diol units. In the partially saponified poly(vinyl alcohols) the ester or the ether group can be distributed statistically or block-wise. Preferred partially saponified poly(vinyl alcohols) used in the context of the present invention have a viscosity of a 4 % aqueous solution at 20°C of 4 to 60 mPa·s, preferably of 4 to 20 mPa·s and in particular of 4 to 10 mPa·s.

Poly(vinyl alcohols) preferred in the context of the present invention are commercially available e. g. under the tradename Mowiol. Those products are characterised by two appended numbers, meaning the viscosity and the degree of saponification. For example, Mowiol 8 - 88 or Mowiol 8/88 mean a poly(vinyl alcohol) having as 4 % aqueous solution at 20°C a viscosity of ca 8 mPa·s and a degree of saponification of 88 mol-%. It is further preferred to use a mixture of two or more compounds. Preferably poly(vinyl alcohols) differing in viscosity as defined above and/or in saponification degree are combined. Particularly preferred are mixture of poly(vinyl alcohols) that differ in viscosity of their 4 % aqueous solutions at 20°C for at least 2 mPa·s or that differ in saponification degree for at least 5 mol-%. Most preferred are mixtures comprising at least 3 types of poly(vinyl alcohols), wherein at least two compounds differ in viscosity as defined above for at least 2 mPa·s and at least two compounds differ in saponification degree for at least 5 mol-%.

Preferably the overall mean saponification degree of all poly(vinyl alcohols) used in the protective layer has to be less than 93 mol-%. In the context of the present invention it is further preferred, that said overall mean saponification degree ranges from 71 mol-% to less than 93 mol-% and in particular from 80 mol-% to 92,9 mol-%.

The overall mean saponification degree of the poly(vinyl alcohols) used in the protective overcoat of a printing plate precursor can be determined experimentally via ¹³C-NMR. To measure the ¹³C-NMR spectra, approximately 200 mg of the protective overcoat are dissolved in 1.0 ml DMSO and from this solution a 75 MHz ¹³C-NMR spectrum is taken, whose resonances can easily be interpreted and allow to calculate the degree of saponification (experimental values). A good correlation is obtained between said experimental values and the values known from the product specification of the poly(vinyl alcohols). The latter values are hereinafter called theoretical values of the mean saponification degree and can easily be calculated, when mixture of poly(vinyl alcohols) are used.

Preferably the poly(vinyl alcohol) is used in 50 to 99.9 weight percent (wt.%) relative to the total weight of the non-volatile compounds of the protective overcoat. Additionally other water soluble polymers can be added to the layer such as poly(vinyl pyrrolidone), poly(ethylene oxide), gelatin, gum arabic, oxygen binding polymers with aliphatic amine groups known from EP 352 630 B1, methyl vinylether/maleic anhydride copolymers, poly(carboxylic acids), copolymers of ethylene oxide and poly(vinyl alcohol), carbon hydrates, hydroxy ethyl cellulose, acidic cellulose, cellulose, poly(arylic acid) and mixtures of these polymers.

Preferably the poly(vinyl pyrrolidone) is only used in small quantities compared to the poly(vinyl alcohol), in particular poly(vinyl pyrrolidone) is used from 0 to 10 parts by weight of the poly(vinyl alcohol) used, from 0 to 3 parts by weight being particularly preferred. Most preferred no poly(vinyl pyrrolidone)compounds are used.

In addition to the poly(vinyl alcohol) and the optional water-soluble polymers disclosed above, the known ingredients of protective layers can be used.

The protective layer has to be transparent for actinic light and preferably has a dry thickness of 0.2 to 10 g/m², 1.0 to 5 g/m² being particularly preferred. Preferably it is homogeneous, substantially impermeable to oxygen, waterpermeable, and can be washed off preferably with the conventional developer solutions used to form a printing relief after imagewise exposure of the photosensitive layer. Said photopolymerizable layer is removed imagewise, whereas the protective layer is removable over the entire area of the element created. The wash-off of the protective layer can be done in a separate step, but can be done during the development step as well.

The protective layer can be coated on the photosensitive layer with known techniques and the coating solution preferably contains water or a mixture of water and an organic solvent. To allow a better wetting, the coating solution preferably contains, related to the solid content, up to 10 wt.%, and particular preferred up to 5 wt.% of a surface active agent. Suitable representatives of surface active agents comprise anionic, cationic and nonionic surface active agents like sodium alkylsulfates and -sulfonates having 12 to 18 carbon atoms, an example of which is sodium dodecylsulfate, N-cetyl- and C-cetyl betaine, alkylaminocarboxylate and -dicarboxylate, and polyethylene glycols with a mean molar weight up to 400.

In addition, further functions can be added to the protective layer. For example, it can be possible to improve the safelight suitability without decreasing the sensitivity of the layer by adding a coloring agent, e. g. a water-soluble dye, that has excellent transmission to the light having a wavelength of 300 to 450 nm and that absorbs the light having a wavelength of 500 nm or more. This principle can easily be varied for different wavelengths to adjust the effective spectral sensitivity distribution of the printing plate precursor as needed.

The photopolymerizable composition comprising the divinylfluorene compound of the present invention can also be used in a method of making a lithographic printing plate comprising the steps of providing a photopolymer printing plate precursor comprising said composition, exposing said printing plate precursor with light comprising radiation in the wavelength range from 300 to 450 nm, preferably with a laser having an emission wavelength in the range from 300 to 450 nm, and processing the printing plate precursor in an aqueous alkaline developer.

In such a process the exposure is done with light comprising radiation in the wavelength range from 350 to 430 nm, preferably from 380 to 430 nm, in particular from 390 to 420 nm and preferably the exposure is done with a laser having an emission wavelenth in the range from 350 to 430 nm, preferably from 380 to 430 nm, in particular in the range from 390 to 420 nm, and the exposure is carried out at an energy density, measured on the surface of the plate, of 100 µJ/cm² or less and preferably of 80 µJ/cm² or less.

The processing of the printing plate precursor comprising the divinylfluorene compound of the present invention is done in the usual manner. After image-wise exposure a pre-heat step is performed to improve the crosslinking of the photosensitive layer. Usually the pre-heat step is then followed by the development step, wherein the complete overcoat layer and the unexposed part of the photosensitive layer are removed. The removal (wash-off) of the overcoat layer and the development of the photosensitive layer can be done in two separate steps in this order, but can also be done in one step simultaneously. Preferably the overcoat layer is washed-off with water before the development step. What remains on the support after the development step are the exposed and thereby photopolymerized parts of the photosensitive layer. The developer solution used for the development of the exposed printing plate precursors preferably is an aqueous alkaline solution having a pH of at least 11, a pH from 11.5 to 13.5 being particularly preferred. The developer solution can contain a small percentage, preferably less than 5 wt.%, of an organic, water-miscible solvent. To adjust the pH of the solution, an alkali hydroxide is preferably used.

Examples of preferred, additional ingredients of the developer solution comprise alone or in combination alkali phosphates, alkali carbonates, alkali bicarbonates, an organic amine compound, alkali silicates, buffering agents, complexants, defoamers, surface active agents and dyes, but the suitable ingredients are not limited to the preferred examples and further ingredients can be used.

The method of development employed is not particularly limited, and may be conducted by soaking and shaking the plate in a developer, physically removing non-image portions while being dissolved in a developer by means of e. g. a brush, or spraying a developer onto the plate so as to remove non-image portions. The time for development is selected depending upon the above method used so that the non-image portions can adequately by removed, and is optionally selected within a range of 5 seconds to 10 minutes.

After the development, the plate my be subjected to a hydrophilic treatment by means of, e. g., gum arabic optionally applied to the printing plate as the case requires (gumming step).

The divinylfluorene compounds of the present invention are stable compounds that usually absorb strongly in the long UV range at about 380 to 390 nm and emit blue fluorescence with a high quantum yield and therefore are good optical brighteners.

In particular those compounds of formulae (II) and (III) that comprise polymerizable substituents, e.g. comprising double or triple bonds or phenolic functions, can be polymerized or copolymerized by known synthetic methods, such as those disclosed in US2003/0091859 to light emitting materials of electroluminescent elements like those disclosed in GB 2 313 127 under formula (I) and US2003/0091859. Therefore the compounds of formulae (II) and (III) of the present invention make possible a new synthetic route to such light emitting materials and also provides access to new polymeric light emitting materials.

### EXAMPLES

### A. Preparation (coating) of the photosensitive layer

A composition was prepared (pw = parts per weight; wt.% = weight percentage) by mixing the components as specified in table 4. This composition was divided equally into 10 portions, and to each portion was added an amount of sensitizer according to table 5. The resulting composition was coated on an electrochemically roughened and anodically oxidized aluminum sheet, the surface of which has been rendered hydrophilic by treatment with an aqueous solution of polyvinyl phosphonic acid (oxide weight 3 g/m²) and was dried for 1 minute at 120°C (circulation oven). The resulting thickness of the layer was 1.5 g/m².

**Table 4**

| Component | Parts per weight (g) |
|---|---|
| a solution containing 32.4 wt.% of a methacrylate/ methacrylic acid copolymer (ratio methylmeth-acrylate: methacrylic acid of 4:1 by weight; acid number: 110 mg KOH/g) in 2-butanone (viscosity 105 mm²/s at 25°C). | 16.075 |
| a solution containing 88.2 wt.% of a reaction product from 1 mole of 2,2,4-trimethyl-hexa-methylenediisocyanate and 2 moles of hydroxyethyl-methacrylate (viscosity 3.30 mm²/s at 25°C) | 14.538 |
| Heliogene blue D 7490® dispersion (9.9 wt.%, viscosity 7.0 mm²/s at 25 °C), trade name of BASF AG | 17.900 |
| 2,2'-bis(2-chlorophenyl)-4,4',5,5'-tetraphenyl-1,2-bisimidazole | 1.448 |
| 2-mercaptobenzothiazole | 0.068 |
| Edaplan LA 411® (1 % in Dowanol PM®, trade mark of Dow Chemical Company) | 2.250 |
| 2-butanone | 78.538 |
| propyleneglycol-monomethylether (Dowanol PM®, trade mark of Dow Chemical Company) | 130.358 |

**Table 5**

| Experiment | Sensitizer | Amount of sensitizer(mmol) |
|---|---|---|
| A | 1,4-di(3,4,5-trimethoxystyryl) benzene | 0.175 |
| B | 1,9-di[3,5-dimethoxy-4-(1-methylpropoxy)styryl]benzene | 0.175 |
| C | 4,4'-di[3,5-dimethoxy-9-(1-methylpropoxy)styryl]biphenyl | 0.175 |
| D | 1,4-di[3,5-dimethoxy-9-(1-methylpropoxy)styryl]-2,3,5,6-tetrachlorobenzene | 0.175 |
| E | (II-1) | 0.175 |
| F | (II-2) | 0.175 |
| G | (II-5) | 0.175 |

On top of the photosensitive layer a solution in water with the composition as defined in table 6 was coated and was dried at 110°C for 2 minutes.

**Table 6**

| Component | Parts by Weight (g) |
|---|---|
| partially hydrolyzed polyvinylalcohol (degree of hydrolysis 88 %, viscosity 4 mPa·s in a solution of 4 wt.% at 20 °C). | 17.03 |
| partially hydrolyzed polyvinylalcohol (degree of hydrolysis 88 %, viscosity 8 mPa·s in a solution of 4 wt.% at 20 °C). | 7.43 |
| fully hydrolyzed polyvinylalcohol (degree of hydrolysis 98 %, viscosity 6 mPa·s in a solution of 4 wt.% at 20 °C). | 14.87 |
| CA 24 E | 0.26 |
| Metolat FC 355 | 0.38 |
| Lutensol A8 (90%) | 0.032 |
| Water | 960 |

The so formed protective overcoat had a dry thickness of 2.0 g/m².

The imaging was carried out with a Polaris XsV violet platesetter device (flat bed system) equipped with a violet laser diode emitting between 392 and 417 nm. The following imaging conditions were used :
Scanning speed : 1000 m/sec
Variable image plane power : 0 to 10.5 mW
Spot diameter : 20 µm
Addressability : 1270 dpi

After imaging the plate was processed in a Agfa VSP85s processor at a speed of 1.2 m/min. During the processing the plate was first heated to 110°C (pre-heat step), next the protective overcoat was washed off and the photolayer was processed in a water based alkaline developer (Agfa PD91) at 28 °C. After a water rinsing and gumming step the printing plate was ready. A 13-step exposure wedge with density increments of 0.15 was used to determine sensitivity of the plate. The results of the exposure tests are shown in table 7 as relative values, wherein the sensitivity of the plate prepared with experiment A (material A) was arbitrarily set to 100 %. For example a relative sensitivity of 200 % corresponds to a material that, compared to material A, only needs 50 % of the exposure energy density (µJ/cm²) for a complete hardening of three wedge steps (the coating is considered as being completely hardened when the density of the processed material is at least 97 % of the density of a plate which has been exposed without filter).

**Table 7**

| Experiment | Relative sensitivity (%) | |
|---|---|---|
| A | 100 | comparison |
| B | 103 | comparison |
| B (2) | 99 | comparison |
| C | 77 | comparison |
| D | No image | comparison |
| E | 128 | invention |
| E(2) | 133 | invention |
| F | 141 | invention |
| F(2) | 133 | invention |
| G | 139 | invention |

It can be clearly seen that there is a significant gain in sensitivity when distyrylfluorene-type sensitizers of the present invention are used compared to the sensitizers known from the state of the art. The experiments B, E and F were run twice as B(2), E(2) and F(2). Their results demonstrate the very good reproducibility of the data.

## Claims

1. A divinylfluorene compound according to one of formulae (II) or (III): 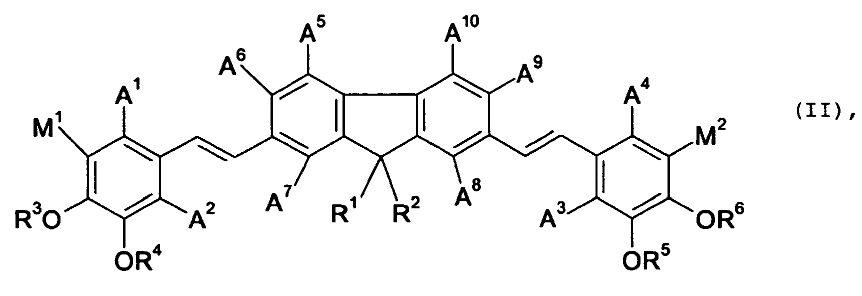 wherein
A¹ to A¹⁰ mutually independent mean a substituent selected from a non-metallic atom group,
M¹ means a hydrogen atom or OR⁷,
M² means a hydrogen atom or OR⁸,
R¹, R² mutually independent mean a substituent selected from a non-metallic atom group, and
R³ to R⁸ mutually independent mean a hydrogen atom, alkyl, an alkyl chain containing double or triple bonds, alkenyl, alkynyl, or any non-metallic atom group linked to the oxygen by a carbon atom,
and wherein one or more pairs of said substituents can jointly mean the remaining atoms to form a ring; or 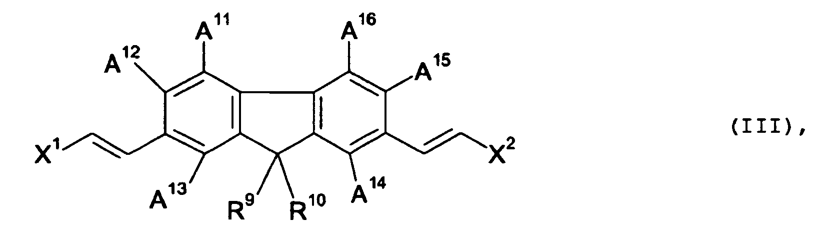
wherein
A¹¹ to A¹⁶ mutually independent mean a substituent selected from a non-metallic atom group,
R⁹, R¹⁰ mutually independent mean a substituent selected from a non-metallic atom group,
X¹, X² mutually independent mean a group of formula (IV) 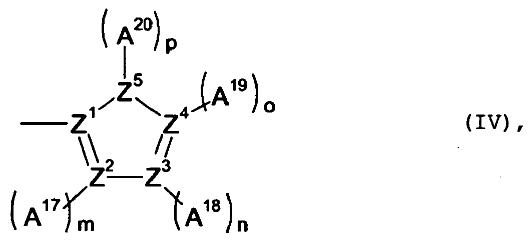
wherein Z¹ to Z⁵ mutually independently mean non-hydrogen, non-metallic atoms,
A¹⁷ to A²⁰ mutually independent mean a substituent selected from a non-metallic atom group,
m, n, o mutually independent mean 0 or 1 and
p means 0, 1 or 2,
and wherein one or more pairs of the substituents in formulae (III) and/or (IV) can jointly mean the remaining atoms to form a ring;
with the proviso that if R⁹, R¹⁰ and A¹¹ to A¹⁶ all mean a hydrogen atom, then X¹ and X² do not both mean an unsubstituted 2-thienyl group and do not both mean an unsubstituted 2-benzoxazolyl-group.

2. A divinylfluorene compound according to claim 1, wherein at least one of the substituents R¹ to R⁸ or at least one of the substituents R⁹ to R¹⁰ is different from a hydrogen atom.

3. A divinylfluorene compound according to any of the preceding claims, wherein A¹ to A¹⁰ or A¹¹ to A¹⁶ mean hydrogen.

4. A divinylfluorene compound according to any of the preceding claims, wherein R¹, R² or R⁹, R¹⁰ mutually independent mean straight chain or branched alkyl having 1 to 20 carbon atoms.

5. A divinylfluorene compound according to any of the preceding claims, wherein the divinylfluorene compound is symmetrical.

6. A divinylfluorene compound according to any of the preceding claims, wherein the divinylfluorene compound is of formula (II).

7. A divinylfluorene compound according to claim 6, wherein R³ to R⁸ mutually independent mean a substituent selected from hydrogen or alkyl.

8. A divinylfluorene compound according to any of claims 1 to 5, wherein the divinylfluorene compound is of formula (III).

9. A divinylfluorene compound according to claim 8, wherein Z¹ to Z⁴ mutually independent mean an unsubstituted nitrogen atom, or a carbon atom that is substituted by a substituent selected from a non-metallic atom group,
Z⁵-(A²⁰)ₚ means O, S, CH₂, CHR¹¹, CR¹²R¹³ or NR¹⁴ and
R¹¹ to R¹⁴ mutually independent mean a substituent selected from a non-metallic atom group.

10. A divinylfluorene compound according to claim 8 or 9, wherein the divinylfluorene compound is of formula (III-A): 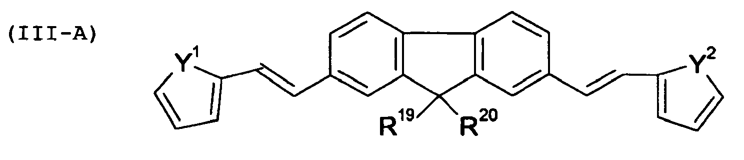 wherein
Y¹, Y² mutually independent mean O, S, NH or N-alkyl,
R¹⁹, R²⁰ mutually independent mean an unsubstituted linear or branched alkyl group having 1 to 20 carbon atoms and wherein the hetero rings comprising Y¹ and Y² are mutually independent each substituted by three members selected from a non-metallic atom group.

11. Method suitable for the synthesis of a divinylfluorene according to any of the preceding claims, **characterized in that** it comprises four steps, wherein in the first step the 9-position of a 2,7,9-unsubstituted fluorene compound is dialkylated using a strong base and an alkyl halide, in step 2 the 9,9-dialkylated fluorene is 2,7-bis-halomethylated, this intermediate is then reacted in step 3 with trialkylphosphite to 2,7-bis(dialkyl-phosphonatomethyl)-9,9-dialkylfluorene, which gives in step 4 on reaction with an aromatic aldehyde 2,7-divinylfluorene compounds.

12. Use of a divinylfluorene compound according to any of claims 1 to 10 as a spectral sensitizer.

13. Use of a divinylfluorene compound according to claim 12 as a sensitizer for a composition that is photopolymerizable upon absorption of light.

14. Use of a divinylfluorene compound according to any of claims 1 to 10 as an optical brightener.

15. Use of a divinylfluorene compound according to any of claims 1 to 10 as a monomer for the synthesis of a polymeric light emitting material for an electroluminescent element.

## Patentansprüche

1. Eine Divinylfluorenverbindung gemäß einer der Formeln (II) oder (III) : 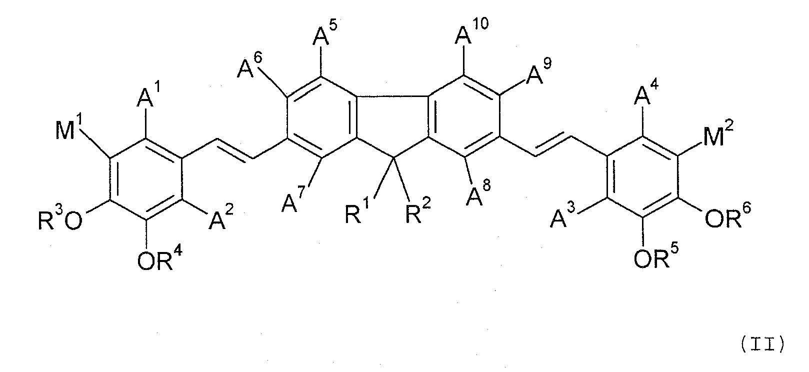 in der :
A¹ bis A¹⁰ unabhängig voneinander jeweils einen Substituenten aus einer Gruppe von Nichtmetallatomen bedeuten,
M¹ ein Wasserstoffatom oder OR⁷ bedeutet,
M² ein Wasserstoffatom oder OR⁸ bedeutet,
R¹ und R² unabhängig voneinander jeweils einen Substituenten aus einer Gruppe von Nichtmetallatomen bedeuten und
R³ bis R⁸ unabhängig voneinander jeweils ein Wasserstoffatom, eine Alkylgruppe, eine Alkylkette mit Doppelbindungen oder Dreifachbindungen, eine Alkenylgruppe, eine Alkynylgruppe oder eine über ein Kohlenstoffatom an das Sauerstoffatom gebundene Nichtmetallatomgruppe bedeuten,
und in der ein oder mehrere Paare der Substituenten gemeinsam die übrigen, zur Bildung eines Ringes benötigten Atome bedeuten können, oder 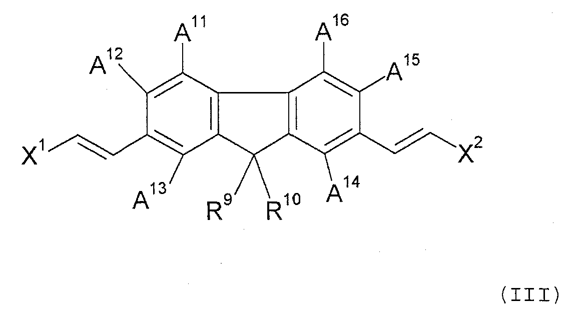
in der :
A¹¹ bis A¹⁶ unabhängig voneinander jeweils einen Substituenten aus einer Gruppe von Nichtmetallatomen bedeuten,
R⁹ und R¹⁰ unabhängig voneinander jeweils einen Substituenten aus einer Gruppe von Nichtmetallatomen bedeuten,
X¹ und X² unabhängig voneinander eine Gruppe der Formel (IV) bedeuten : 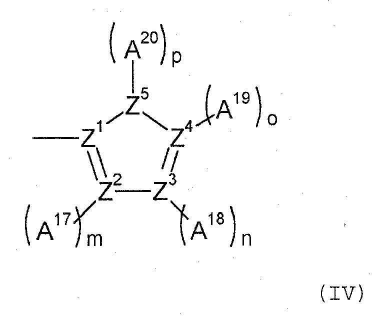
in der :
Z¹ bis Z⁵ unabhängig voneinander jeweils ein beliebiges Atom außer einem Wasserstoffatom oder einem Metallatom bedeuten,
A¹⁷ bis A²⁰ unabhängig voneinander jeweils einen Substituenten aus einer Gruppe von Nichtmetallatomen bedeuten,
m, n und o unabhängig voneinander jeweils 0 oder 1 bedeuten und p 0, 1 oder 2 bedeutet,
und in der ein oder mehrere Paare der Substituenten in den Formeln (III) und/oder (IV) gemeinsam die übrigen, zur Bildung eines Ringes benötigten Atome bedeuten können,
vorausgesetzt, dass wenn R⁹, R¹⁰ und A¹¹ bis A¹⁶ alle ein Wasserstoffatom bedeuten, X¹ und X² nicht beide eine nichtsubstituierte 2-Thienylgruppe und nicht beide eine nichtsubstituierte 2-Benzoxazolylgruppe bedeuten.

2. Eine Divinylfluorenverbindung nach Anspruch 1, **dadurch gekennzeichnet, dass** zumindest einer der Substituenten R¹ bis R⁸ oder zumindest einer der Substituenten R⁹ bis R¹⁰ kein Wasserstoffatom bedeutet.

3. Eine Divinylfluorenverbindung nach einem der vorstehenden Ansprüche, **dadurch gekennzeichnet, dass** A¹ bis A¹⁰ oder A¹¹ bis A¹⁶ ein Wasserstoffatom bedeuten.

4. Eine Divinylfluorenverbindung nach einem der vorstehenden Ansprüche, **dadurch gekennzeichnet, dass** R¹, R² oder R⁹ und R¹⁰ unabhängig voneinander jeweils eine geradkettige oder verzweigtkettige Alkylgruppe mit 1 bis 20 Kohlenstoffatomen bedeuten.

5. Eine Divinylfluorenverbindung nach einem der vorstehenden Ansprüche, **dadurch gekennzeichnet, dass** die Divinylfluorenverbindung symmetrisch ist.

6. Eine Divinylfluorenverbindung nach einem der vorstehenden Ansprüche, **dadurch gekennzeichnet, dass** die Divinylfluorenverbindung der Formel (II) entspricht.

7. Eine Divinylfluorenverbindung nach Anspruch 6, **dadurch gekennzeichnet, dass** R³ bis R⁸ unabhängig voneinander jeweils ein Wasserstoffatom oder eine Alkylgruppe als Substituent bedeuten.

8. Eine Divinylfluorenverbindung nach einem der Ansprüche 1 bis 5, **dadurch gekennzeichnet, dass** die Divinylfluorenverbindung der Formel (III) entspricht.

9. Eine Divinylfluorenverbindung nach Anspruch 8, **dadurch gekennzeichnet, dass** Z¹ bis Z⁴ unabhängig voneinander jeweils ein nicht-substituiertes Stickstoffatom oder ein durch einen Substituenten aus einer Gruppe von Nichtmetallatomen substituiertes Kohlenstoffatom bedeuten, Z⁵-(A²⁰)ₚ O, S, CH₂, CHR¹¹, CR¹²R¹³ oder NR¹⁴ bedeutet und R¹¹ bis R¹⁴ unabhängig voneinander jeweils einen Substituenten aus einer Gruppe von Nichtmetallatomen bedeuten.

10. Eine Divinylfluorenverbindung nach Anspruch 8 oder 9, **dadurch gekennzeichnet, dass** die Divinylfluorenverbindung der Formel (III-A) entspricht : 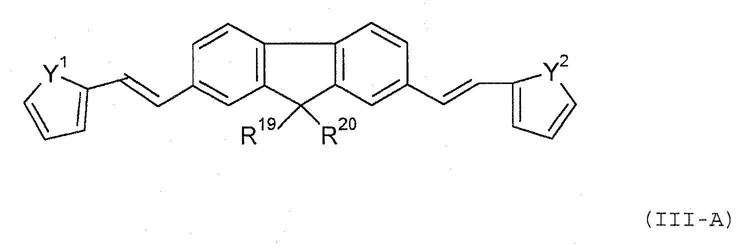 in der :
Y¹ und Y² unabhängig voneinander jeweils O, S, NH oder N-Alkyl bedeuten und
R¹⁹ und R²⁰ unabhängig voneinander jeweils eine nichtsubstituierte, geradkettige oder verzweigtkettige Alkylgruppe mit 1 bis 20 Kohlenstoffatomen bedeuten,
und die Y¹ und Y² enthaltenden Heteroringe unabhängig voneinander jeweils durch drei Substituenten aus einer Gruppe von Nichtmetallatomen substituiert sind.

11. Ein geeignetes Verfahren für die Synthese eines Divinylfluorens nach einem der vorstehenden Ansprüche, **dadurch gekennzeichnet, dass** das Verfahren 4 Schritte umfasst, wobei im ersten Schritt die 9-Stellung einer nicht-2,7,9-substituierten Fluorenverbindung mit einer starken Base und einem Alkylhalogenid dialkyliert und in Schritt 2 das 9,9-dialkylierte Fluoren 2,7-bishalomethyliert wird, wonach man dieses Zwischenprodukt in Schritt 3 mit Trialkylphosphit zu 2,7-Bis-(dialkylphosphonatmethyl)-9,9-dialkylfluoren reagieren lässt, das man sodann in Schritt 4 mit einem aromatischen Aldehyd reagieren lässt, um 2,7-Divinylfluorenverbindungen zu erhalten.

12. Verwendung einer Divinylfluorenverbindung nach einem der Ansprüche 1 bis 10 als spektraler Sensibilisator.

13. Verwendung einer Divinylfluorenverbindung nach Anspruch 12 als Sensibilisator für eine Zusammensetzung, die durch Absorption von Licht fotopolymerisierbar ist.

14. Verwendung einer Divinylfluorenverbindung nach einem der Ansprüche 1 bis 10 als optisches Aufhellmittel.

15. Verwendung einer Divinylfluorenverbindung nach einem der Ansprüche 1 bis 10 als Monomer für die Synthese eines polymeren, lichtemittierenden Materials für ein Elektrolumineszenzelement.

## Revendications

1. Un composé de divinylfluorène répondant à la formule (II) ou à la formule (III) : 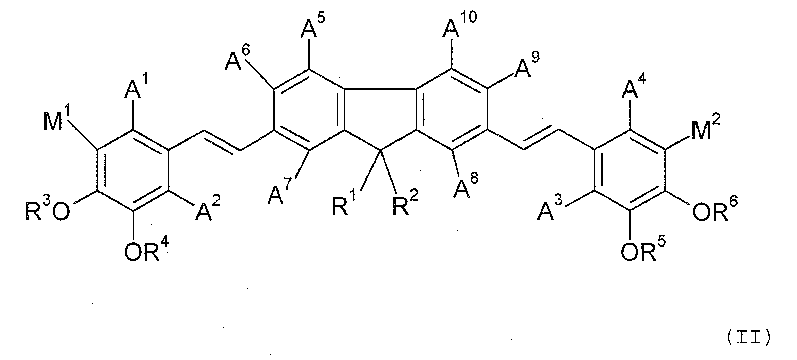 où :
A¹ à A¹⁰ représentent, indépendamment l'un de l'autre, un substituant choisi parmi un groupe d'atomes non métalliques,
M¹ représente un atome d'hydrogène ou OR⁷,
M² représente un atome d'hydrogène ou OR⁸,
R¹ et R² représentent, indépendamment l'un de l'autre, un substituant choisi parmi un groupe d'atomes non métalliques,
R³ à R⁸ représentent, indépendamment l'un de l'autre, un atome d'hydrogène, un groupe alkyle, une chaîne alkyle comprenant des doubles liaisons ou des triples liaisons, un groupe alkényle, un groupe alkynyle ou un groupe d'atomes non métalliques lié à l'atome d'oxygène par un atome de carbone,
et où une ou plusieurs paires desdits substituants peuvent représenter conjointement les autres atomes nécessaires pour la formation d'un noyau, ou 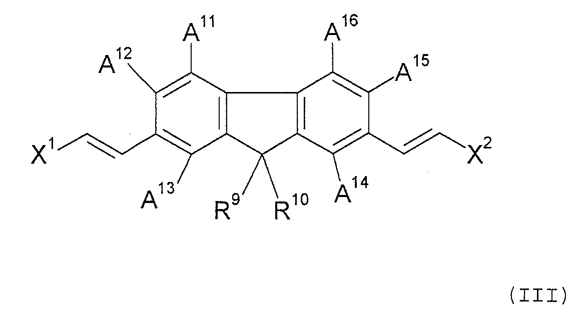
où :
A¹¹ à A¹⁶ représentent, indépendamment l'un de l'autre, un substituant choisi parmi un groupe d'atomes non métalliques,
R⁹ et R¹⁰ représentent, indépendamment l'un de l'autre, un substituant choisi parmi un groupe d'atomes non métalliques,
X¹ et X² représentent, indépendamment l'un de l'autre, un groupe répondant à la formule (IV) : 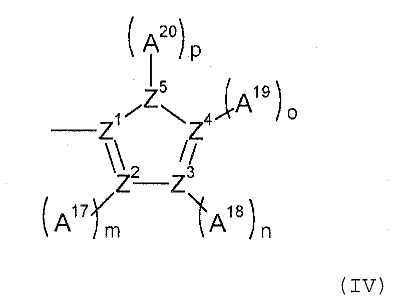
où :
Z¹ à Z⁵ représentent, indépendamment l'un de l'autre, un atome quelconque, excepté un atome d'hydrogène ou un atome métallique,
A¹⁷ à A²⁰ représentent, indépendamment l'un de l'autre, un substituant choisi parmi un groupe d'atomes non métalliques,
m, n et o représentent, indépendamment l'un de l'autre, 0 ou 1 et p représente 0, 1 ou 2,
et où une ou plusieurs paires des substituants dans les formules (III) et/ou (IV) peuvent représenter conjointement les autres atomes nécessaires pour la formation d'un noyau,
à condition que, si R⁹, R¹⁰ et A¹¹ à A¹⁶ représentent tous un atome d'hydrogène, X¹ et X² ne représentent pas tous les deux un groupe 2-thiényle non substitué ni un groupe 2-benzoxazolyle non substitué.

2. Un composé de divinylfluorène selon la revendication 1, **caractérisé en ce qu'**au moins un des substituants R¹ à R⁸ ou au moins un des substituants R⁹ à R¹⁰ n'est pas un atome d'hydrogène.

3. Un composé de divinylfluorène selon l'une quelconque des revendications précédentes, **caractérisé en ce que** A¹ à A¹⁰ ou A¹¹ à A¹⁶ représentent un atome d'hydrogène.

4. Un composé de divinylfluorène selon l'une quelconque des revendications précédentes, **caractérisé en ce que** R¹, R² ou R⁹ et R¹⁰ représentent, indépendamment l'un de l'autre, un groupe alkyle à chaînes droites ou à chaînes ramifiées contenant 1 à 20 atomes de carbone.

5. Un composé de divinylfluorène selon l'une quelconque des revendications précédentes, **caractérisé en ce que** le composé de divinylfluorène est un composé symétrique.

6. Un composé de divinylfluorène selon l'une quelconque des revendications précédentes, **caractérisé en ce que** le composé de divinylfluorène répond à la formule (II).

7. Un composé de divinylfluorène selon la revendication 6, **caractérisé en ce que** R³ à R⁸ représentent, indépendamment l'un de l'autre, un atome d'hydrogène ou un groupe alkyle comme substituant.

8. Un composé de divinylfluorène selon l'une quelconque des revendications 1 à 5, **caractérisé en ce que** le composé de divinylfluorène répond à la formule (III).

9. Un composé de divinylfluorène selon la revendication 8, **caractérisé en ce que** Z¹ à Z⁴ représentent, indépendamment l'un de l'autre, un atome d'azote non substitué ou un atome de carbone substitué par un substituant choisi parmi un groupe d'atomes non métalliques, Z⁵-(A²⁰)ₚ représente O, S, CH₂, CHR¹¹, CR¹²R¹³ ou NR¹⁴ et R¹¹ à R¹⁴ représentent, indépendamment l'un de l'autre, un substituant choisi parmi un groupe d'atomes non métalliques.

10. Un composé de divinylfluorène selon la revendication 8 ou 9,
**caractérisé en ce que** le composé de divinylfluorène répond à la formule (III-A) : 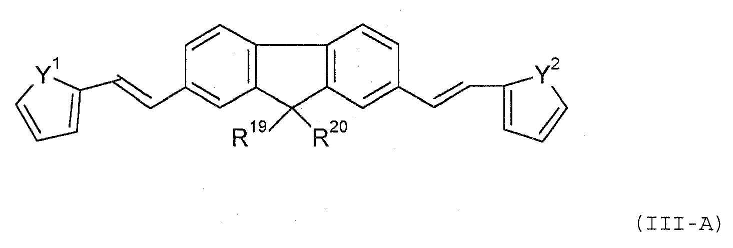 où :
Y¹ et Y² représentent, indépendamment l'un de l'autre, O, S, NH ou N-alkyle et
R¹⁹ et R²⁰ représentent, indépendamment l'un de l'autre, un groupe alkyle non substitué, à chaînes droites ou à chaînes ramifiées, contenant 1 à 20 atomes de carbone,
et où des noyaux hétérocycliques contenant Y¹ et Y² sont substitués, indépendamment l'un de l'autre, par trois substituants choisis parmi un groupe d'atomes non métalliques.

11. Un procédé approprié pour la synthèse d'un divinylfluorène selon l'une quelconque des revendications précédentes, **caractérisé en ce que** le procédé comprend 4 étapes, la première étape comprenant la dialkylation de la position 9 d'un composé de fluorène non 2,7,9-substitué avec une base forte et avec un halogénure d'alkyle, l'étape 2 comprenant la 2,7-bishalométhylation du fluorène 9,9-dialkylé, l'étape 3 consistant à faire réagir ce produit intermédiaire avec du phosphite de trialkyle et l'étape 4 consistant à faire réagir le fluorène de 2,7-bis(dialkylphosphonatométhyl)-9,9-dialkyle ainsi obtenu avec un aldéhyde aromatique, ce qui génère des composés de 2,7-divinylfluorène.

12. L'utilisation d'un composé de divinylfluorène selon l'une quelconque des revendications 1 à 10 comme sensibilisateur spectral.

13. L'utilisation d'un composé de divinylfluorène selon la revendication 12 comme sensibilisateur pour une composition photopolymérisable par absorption de lumière.

14. L'utilisation d'un composé de divinylfluorène selon l'une quelconque des revendications 1 à 10 comme agent de blanchiment optique.

15. L'utilisation d'un composé de divinylfluorène selon l'une quelconque des revendications 1 à 10 comme monomère pour la synthèse d'un matériau polymère luminescent pour un élément électroluminescent.
